# EUROPEAN PATENT APPLICATION

(11) **EP 2 870 948 A1**
(43) Date of publication of application: **13.05.2015**
(21) Application number: 13192203.1
(22) Date of filing: 08.11.2013
(51) Int. Cl.: A61F 5/02

(54) **Posture loop**

(71) Applicant: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(57) **Abstract**

A posture loop includes an cable (10) having two ends (11, 12) secured to form an endless loop, and a bridge (5) engaged between two segments (14, 15) of the cable (10) for forming an eight (8)-shaped structure for the posture loop and for engaging onto shoulder (80) of a user (8), a buckle (2) is secured to the first end (11) of the cable (10), and a latch (25) is secured to the second end (12) of the cable (10), and the latch (25) is engageable with the buckle (2) for securing the first end (11) and the second end (12) of the cable (10) together in order to form an endless loop.

## Description

The invention relates to a posture loop for straightening the shoulder and/or the back of the user.

Typical elastic exercise devices comprise an elastic cable for exercising purposes, but may not be used to straighten the shoulder and/or the back of the user.

The invention provides a posture loop as defined in the claims.

In the drawing:
Fig. 1 is a rear view of a posture loopaccording to an embodiment of the invention;
Figs. 2, 3 are partial perspective views of the posture loop;
Fig. 4 is a partial exploded view of the posture loop;
Fig. 5 is a plan view of the posture loop; and
Figs. 6, 7 are partial cross sectional views of the posture loop.

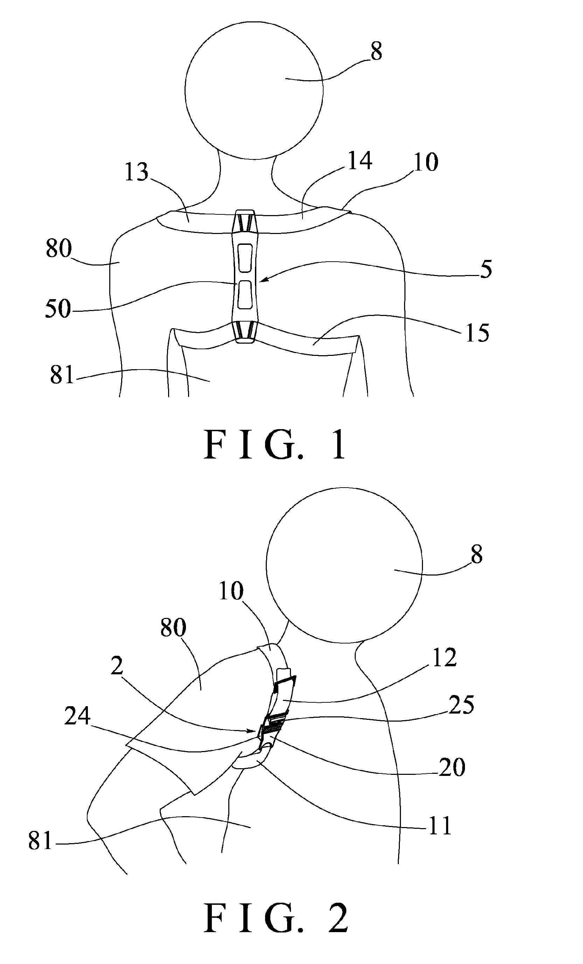

Referring to Figs. 1-5, a posture loop comprises an elastic cable 10 having two ends 11, 12 coupled with a buckle 2, and a middle portion 13 coupled together with a retainer bridge 5 for forming an eight (8)-shaped structure and for engaging with and for straightening the shoulder 80 and/or the back 81 of a user 8 (Figs. 1-3).

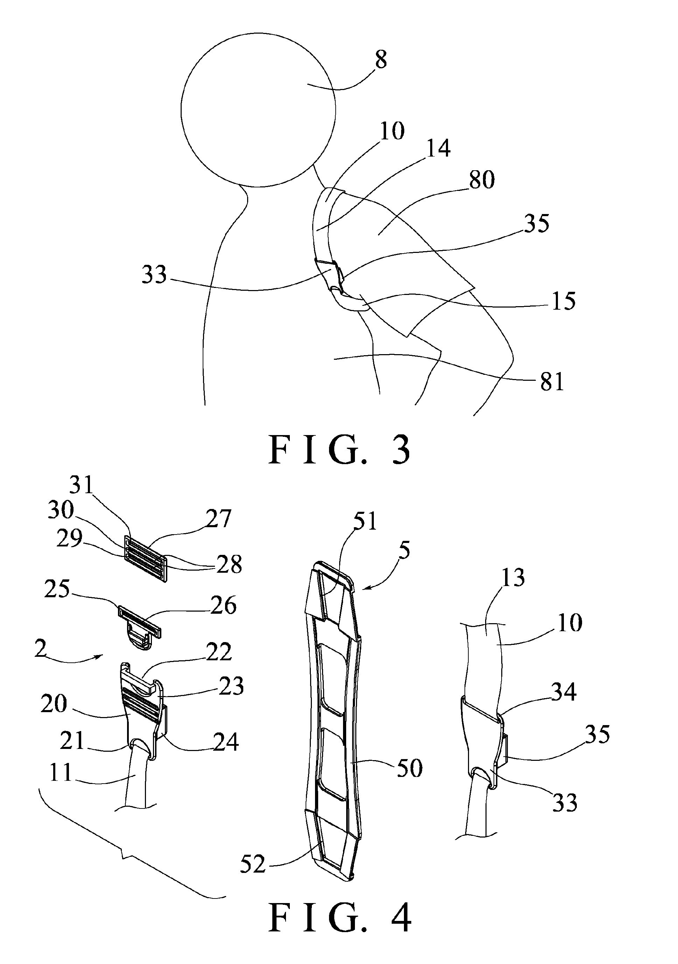

The buckle 2 includes a female holder 20 (Figs. 4-7) having a first end 21 coupled to the first end 11 of the cable 10, and having a latch opening 22 formed on the second end 23 of the holder 20 for engaging with a male latch 25. The holder 20 includes a resilient cushion 24 for softly engaging with the user 8. The latch 25 includes a channel 26 for attaching the second end 12 of the cable 10. A latch ring 27 includes two bars 28 for forming three grooves 29, 30, 31 and for engaging with the cable 10.

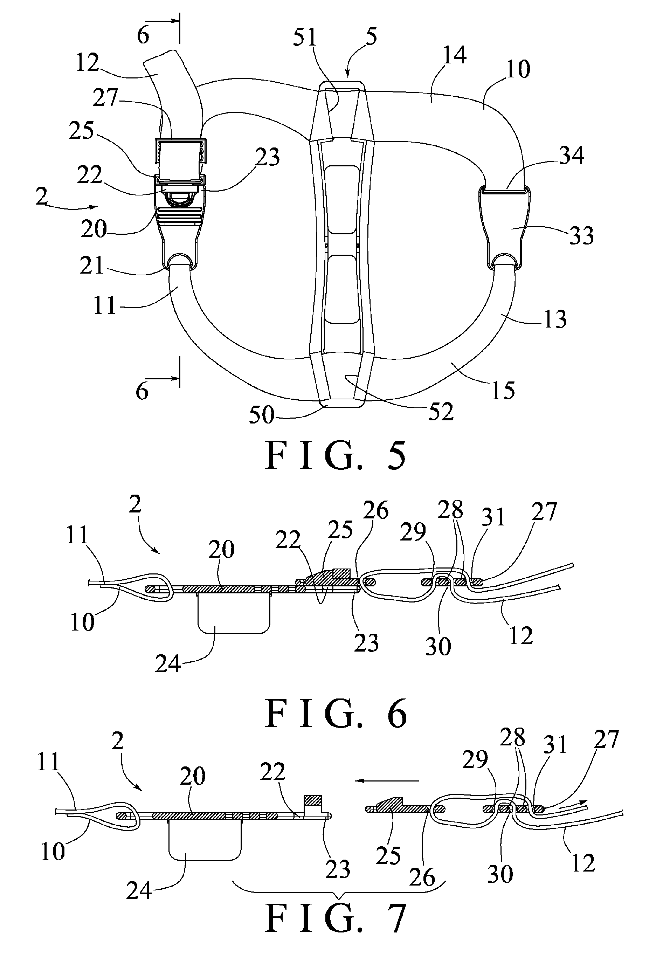

The second end 12 of the cable 10 is engaged through one of the grooves 30 (Figs. 6-7) and over one of the bars 28 and through the other groove 29 of the ring 27 and then through the channel 26 of the latch 25 and the further groove 31 of the ring 27 for adjustably coupling the second end 12 of the cable 10 to the latch 25 and then to the holder 20, and for adjusting the length of the endless loop of the cable 10.

A pad 33 includes a passage 34 for engaging with the middle portion 13 of the cable 10, and includes a cushion 35 for softly engaging with the user 8. The middle portion 13 of the cable 10 is spaced by the holder 20 and the pad 33 into a first segment 14 and a second segment 15. The retainer bridge 5 is disposed between the holder 20 and the pad 33 and between the segments 14, 15 of the cable 10 for forming an eight (8)-shaped structure, and includes two pathways 51, 52 for engaging with the segments 14, 15 of the cable 10 for spacing the segments 14, 15 from each other.

In operation, the posture loop is engageable onto the shoulder 80 of the user 8 (Figs. 1-3) for straightening the shoulder 80 and/or the back 81 of the user 8. The second end 12 of the cable 10 is adjustable relative to the latch 25 (Figs. 5-7) for adjusting the length of the cable 10 and for engaging with the users 8 of different dimensions.

## Claims

1. A posture loop comprising an cable (10) including a first end (11) and a second end (12), **characterised in that** the cable (10) includes a middle portion (13) separated into a first segment (14) and a second segment (15), and a bridge (5) is engaged between the first and the second segments (14, 15) of the cable (10), a buckle (2) is secured to the first end (11) of the cable (10), and a latch (25) is secured to the second end (12) of the cable (10), and the latch (25) is engageable with the buckle (2).

2. The posture loop as claimed in claim 1, **characterised in that** the bridge (5) includes an upper pathway (51) and a lower pathway (52) for engaging with the first segment (14) and the second segment of the cable (10).

3. The posture loop as claimed in claim 1 or 2, **characterised in that** the buckle (2) includes a cushion (24).

4. The posture loop as claimed in one of claims 1 to 3, **characterised in that** the buckle (2) includes a latch opening (22) for engaging with the latch (25).

5. The posture loop as claimed in one of claims 1 to 4, **characterised in that** the latch (25) includes a channel (26) for engaging with the second end (12) of the cable (10).

6. The posture loop as claimed in one of claims 1 to 5, **characterised in that** a ring (27) is engaged with the second end (12) of the cable (10).

7. The posture loop as claimed in claim 6, **characterised in that** the ring (27) has at least one bar (28) for forming at least one groove (29, 30, 31).

8. The posture loop as claimed in one of claims 1 to 7, **characterised in that** a pad (33) is attached to the middle portion (13) of the cable (10).
